(19) **Europäisches Patentamt / European Patent Office / Office européen des brevets**

(11) **EP 1 628 646 B1**

(12) **FASCICULE DE BREVET EUROPEEN**

(45) Date de publication et mention de la délivrance du brevet:
**14.07.2010 Bulletin 2010/28**

(51) Int Cl.:
*A61K 31/00* (2006.01)  *A01N 1/00* (2006.01)
*A61P 31/00* (2006.01)

(21) Numéro de dépôt: **04742585.5**

(22) Date de dépôt: **27.04.2004**

(86) Numéro de dépôt international:
**PCT/FR2004/001018**

(87) Numéro de publication internationale:
**WO 2004/096195 (11.11.2004 Gazette 2004/46)**

(54) **UTILISATION D'AGONISTES DE PPAR-GAMMA COMME AGENTS ANTI-INFECTIEUX**

VERWENDUNG VON PPAR-GAMMA-AGONISTEN ALS MITTEL GEGEN INFEKTIONEN

USE OF PPAR-GAMMA AGONISTS AS ANTI-INFECTIVE AGENTS

(84) Etats contractants désignés:
**CH DE FR GB LI**

(30) Priorité: **28.04.2003 FR 0305188**

(43) Date de publication de la demande:
**01.03.2006 Bulletin 2006/09**

(73) Titulaire: **UNIVERSITE PAUL SABATIER TOULOUSE III**
**31062 Toulouse Cédex 4 (FR)**

(72) Inventeurs:
• **COSTE, Agnès**
  **F-31700 Blagnac (FR)**
• **PIPY, Bernard**
  **F-31400 Toulouse (FR)**

(74) Mandataire: **Cabinet BARRE LAFORGUE & associés**
**95, rue des Amidonniers**
**31000 Toulouse (FR)**

(56) Documents cités:
| | |
|---|---|
| WO-A-01/28532 | WO-A-98/57631 |
| WO-A-02/080956 | WO-A-02/089790 |
| WO-A-03/059271 | GB-A- 2 373 725 |

• HAYES M. M. ET AL.: "Peroxisome proliferator-activated receptor gamma agonists inhibit HIV-1 replication in macrophages by transcriptional and post-transcriptional effects." J. BIOL. CHEM., vol. 277, no. 19, 10 mai 2002 (2002-05-10), pages 16913-16919, XP002275557
• SKOLNIK P.R. ET AL.: "Stimulation of peroxisome proliferator-activated receptors alpha and gamma blocks HIV-1 production in acutely infected primary blood cells, chronically infected U1 cells, alveolar macrophages from HIV-infected subjects." JAIDS, vol. 31, 1 septembre 2002 (2002-09-01), pages 1-10, XP009028634
• PICA F ET AL: "12-PROSTAGLANDIN J2 IS A POTENT INHIBITOR OF INFLUENZA A VIRUS REPLICATION" ANTIMICROBIAL AGENTS AND CHEMOTHERAPY, AMERICAN SOCIETY FOR MICROBIOLOGY, WASHINGTON, DC, US, vol. 44, no. 1, janvier 2000 (2000-01), pages 200-204, XP002909741 ISSN: 0066-4804
• VAMECQ J ET AL: "Medical significance of peroxisome proliferator-activated receptors" LANCET, XX, XX, vol. 354, no. 9173, 10 juillet 1999 (1999-07-10), pages 141-148, XP004266618 ISSN: 0140-6736

EP 1 628 646 B1

**Description**

L'invention concerne un procédé de fabrication d'une composition thérapeutique, stimulant la réponse immunitaire innée par induction et/ou amplification de l'expression des récepteurs mannose des macrophages. L'invention s'inscrit avantageusement dans le cadre des traitements préventifs ou curatifs de maladies infectieuses d'origine bactérienne, fongique, protozoaire... et présente un intérêt tout particulier, dans la médication de sujets immunodéprimés.

La réponse immunitaire innée, essentielle dans les mécanismes de défense de l'organisme contre les agents infectieux, est une réaction immune précoce qui se déclenche dès les premiers instants de l'infection. Les macrophages participent à cette réponse immunitaire innée en reconnaissant, en phagocytant et en détruisant l'agent infectieux. La phase de reconnaissance consiste en l'identification de structures biochimiques superficielles caractéristiques des pathogènes et fait intervenir nombre de récepteurs à la surface des macrophages.

Parmi ces récepteurs, le récepteur mannose (on parle aussi de récepteur pour le mannose) permet la reconnaissance, mais également la fixation et la phagocytose, d'une grande diversité de pathogènes, en particulier des pathogènes d'origine bactérienne, fongique et protozoaire, qui présentent sur la face extérieure de leurs parois des glycoprotéines, des polysaccharides et/ou des lipopolysaccharides ayant à leurs extrémités libres des motifs mannosylés et/ou fucosylés. A des fins de simplification, dans le reste du texte, ces pathogènes seront désignés par "pathogènes à motifs mannosylés et/ou fucosylés".

L'implication du récepteur mannose des macrophages (MMR) est ainsi essentielle dans les processus de défense de l'organisme. Elle n'est toutefois effective que si ce récepteur est fortement exprimé à la surface des macrophages.

Cependant, bien que le rôle du récepteur mannose des macrophages soit aujourd'hui bien défini, les voies de régulation de l'expression de ce récepteur restent encore mal connues. Il en est de même pour les acteurs biochimiques intervenant dans l'induction de l'expression de ce récepteur.

Dans ce contexte, l'invention a pour principal objectif de proposer des composés capables d'induire et/ou d'amplifier l'expression des MMR. Elle vise ainsi à mettre en évidence des composés capables d'améliorer la vigilance et/ou la réponse du système immunitaire, à l'égard de nombreux micro-organismes via une reconnaissance par les récepteurs mannose des macrophages.

Dans tout le texte, on entend par "milieu biologique", un milieu de composition biologique, c'est-à-dire comprenant de la matière vivante, notamment des organismes monocellulaires et/ou pluricellulaires. Un milieu biologique peut être artificiel (par exemple, un milieu de culture), ou naturel (par exemple, un tissu vivant, une composition sanguine, isolés ou non d'un organisme pluricellulaire -notamment un-primate-).

Par ailleurs, on désigne par "macrophage", toute cellule, de localisation essentiellement tissulaire, issue de la différenciation des monocytes (les macrophages en tant que tels, les cellules dendritiques, les cellules neutrophiles phagocytaires) ainsi que les monocytes. "Macrophage" au sens de l'invention désigne ainsi, de manière générale, toute cellule susceptible d'exprimer le MMR et ayant la capacité à phagocyter.

L'invention vise également à proposer de nouvelles perspectives dans le traitement thérapeutique de maladies infectieuses -notamment des maladies opportunistes-, en particulier dans le cadre de la médication de sujets immunodéprimés.

L'invention concerne un procédé selon la revendication 1.

L'invention concerne donc, en premier lieu, un procédé de stimulation de l'expression de récepteurs mannose de macrophages dans lequel, on traite, par une quantité efficace d'au moins un ligand agoniste de PPARγ, un milieu biologique, extracorporel ou intracorporel, comprenant des macrophages. On entend par "quantité efficace", une quantité apte à induire et/ou à amplifier l'expression des récepteurs mannose d'au moins une partie des macrophages du milieu biologique cible.

L'invention repose ainsi sur la constatation, surprenante et jusqu'alors insoupçonnée, que l'activation du récepteur nucléaire PPARγ conduit à une induction génique et à une expression protéique du MMR.

D'après Vamecq J. et Latruffe N., 1999 (The Lancet, 354:141-148), un document récapitulatif des connaissances générales concernant les PPARs (récepteurs des proliférateurs de peroxisomes), PPARγ serait impliqué dans des phénomènes d'adipogénèse, de différenciation cellulaire et de régulation de certaines réactions anti-inflammatoires. Bien qu'étant essentiellement exprimé dans les cellules adipeuses, PPARγ a également été détecté dans les macrophages.

Les MMR ne sont pas abordés dans cette publication.

Ainsi, jusqu'à présent, rien ne permettait de soupçonner une quelconque implication de PPARγ dans les mécanismes de stimulation et/ou d'amplification de l'expression des MMR.

La mise en évidence par les inventeurs du lien fonctionnel existant entre le facteur de transcription PPARγ et l'expression du MMR, ouvre ainsi de nouvelles perspectives d'application aux ligands agonistes de PPARγ.

Avantageusement, au moins un des ligands agonistes de PPARγ utilisés pour induire et/ou amplifier l'expression du MMR selon l'invention, est choisi parmi le groupe des ligands naturels : la 15d-PGJ$_2$, les prostaglandines A1 et D2, les acides 9- et 13-hydoxyoctadécanoïques, les dérivés des LDL (lipoprotéines de faible densité) oxydées.

Parmi ces ligands naturels, la 15d-PGJ$_2$ (15-déoxy-$\Delta^{12,14}$-prostaglandine J$_2$), un métabolite de la prostaglandine D2 (PGD2), est connue comme étant un des plus puissants ligands agonistes de PPARγ.

On peut également et avantageusement choisir au moins un des ligands agonistes de PPARγ parmi le groupe des ligands synthétiques : la troglitazone (Resulin®), la rosiglitazone (Avandia®), la pioglitazone (Actos®), la ciglitazone, l'englitazone, le GW 347845, le KRP-297, le JTT-501B, le SB 213068, le GI 262570, le GW 1929, le GW 7845, le GW 0207, le L-796449, l'indométhacine, l'ibuprofène.

La troglitazone, la rosiglitazone, la pioglitazone, la ciglitazone, l'englitazone, le GW 347845 appartiennent à la famille des thiazolidinediones et sont couramment utilisés dans le domaine médical comme médicaments hypolipidémiants pour le traitement de certains diabètes. De même, l'indométhacine et l'ibuprofène sont communément utilisés comme anti-inflammatoires non-stéroïdiens pour le traitement de certaines maladies inflammatoires, comme l'athérosclérose et l'arthrose.

En particulier, WO 03/059271 enseigne l'utilisation des composés agonistes de PPARγ pour traiter ou pour prévenir les inflammations, les cancers, les maladies cardiovasculaires... WO 03/059271 n'aborde pas les MMR et pour ce qui est des méthodes et des compositions thérapeutiques décrites, elles ne sont envisagées que comme remèdes aux symptômes de certaines inflammations. Il n'est décrit ni suggéré un quelconque effet biologique des ligands agonistes de PPARγ sur les causes de ces inflammations, notamment sur d'éventuels agents infectieux susceptibles-de générer certaines de ces réactions inflammatoires.

Ainsi, jamais encore, il n'a été envisagé d'utiliser ces mêmes composés pour stimuler et/ou amplifier l'expression des récepteurs mannose des macrophages et encore moins pour obtenir le déclenchement des fonctions effectrices des macrophages (phagocytose, synthèse des cytokines et des agents oxydants, présentation de l'antigène) contre des pathogènes d'origine bactérienne, fongique ou protozoaire.

En utilisant *Candida albicans* (*C. albicans*) comme modèle de micro-organisme sensible au MMR, les inventeurs ont pourtant pu déterminer que, au-delà d'une simple modulation de l'expression du MMR, le traitement des macrophages par un ligand agoniste de PPARγ conduit à une stimulation remarquable de la capacité des macrophages à reconnaître et à éliminer l'agent pathogène, non seulement par phagocytose, mais également par une production accrue d'agents oxydants.

Les inventeurs sont ainsi parvenus à démontrer que l'induction du récepteur mannose par les ligands agonistes de PPARγ permet l'élimination d'une infection fongique du tractus gastro-intestinal par *C. albicans*. Cette élimination se fait en absence d'anticorps ou d'une réponse immune adaptative. L'effet anti-infectieux des ligands agonistes de PPARγ est la conséquence, d'une part, de l'accroissement de la phagocytose de *C. albicans* médiée par le récepteur mannose et, d'autre part, de la mort de *C. albicans* causée par les intermédiaires réactifs de l'oxygène (IROs). Ces derniers sont sécrétés par les macrophages au cours de la phagocytose de la levure. En effet, il a été démontré que la fonction candidicide des macrophages disparaît lorsque l'anion superoxyde est supprimé par la superoxyde dismutase (SOD) et que la production des espèces réactives de l'oxygène par les macrophages est supprimée par les antisens des ARN messagers codant pour le MMR (Coste A. et al., Immunity, 2003, 19:329-339). Ces enseignements qui découlent des travaux des inventeurs, ainsi que l'invention, sont totalement indépendants des propriétés anti-inflammatoires de PPARγ.

Cette capacité qu'ont les ligands agonistes de PPARγ à stimuler l'expression des récepteurs mannose et donc, d'une part, à renforcer la vigilance des macrophages à l'égard des micro-organismes infectieux à motifs mannosylés et/ou fucosylés et, d'autre part, à déclencher les-fonctions effectrices des macrophages, lorsque ceux-ci se trouvent en présence de ce type de pathogènes, peut être avantageusement mise à profit pour la médication de sujets atteints de déficits immunitaires, innés ou acquis.

Pour certains sujets immunodéprimés, comme les sidéens ou les patients traités par chimiothérapie (par exemple, des patients atteints d'un cancer ou d'une hémopathie) ou par des immunosuppresseurs (par exemple, après une transplantation), l'induction et/ou l'amplification de l'expression MMR permettrait de pallier efficacement l'insuffisance de leur système immunitaire, en améliorant non seulement la vigilance du système immunitaire innée à l'égard de nombreux micro-organismes infectieux, mais également en stimulant et en amplifiant les fonctions effectrices des macrophages en vue d'obtenir une réponse immune efficace, de type Th2. Eventuellement, l'induction et/ou l'amplification de l'expression des MMR va(vont) permettre de réorienter la défense immunitaire vers une réponse de type Th2 pour compenser une réponse de type Th1 éventuellement défectueuse.

Dans ce contexte de stimulation du système immunitaire via l'expression des MMR, l'invention s'adresse en particulier à des sujets immunodéprimés. Elle s'adresse également à des personnes susceptibles d'être exposées à des agents infectieux présentant sur la face extérieure de leurs parois des motifs mannosylés et/ou fucosylés, ou ayant été infectées par ces derniers. A titre d'exemples d'agents infectieux visés par l'invention, on peut citer : *Streptococcus pneumoniae, Klebsellia pneumoniae, Cryptococcus neoformans, Mycobacterium tuberculosis, Candida albicans, Leishmania donovani, Pneumocystis carinii, Trypanosoma cruzi, Aspergillus fumigatus, pseudomonas aeruginosas, Legionella pneumophilla*.

La stimulation du système immunitaire d'un individu -en particulier, d'un individu immunodéprimé-, par amplification de l'expression du MMR, peut être réalisée en administrant à celui-ci une quantité efficace d'au moins un ligand agoniste de PPARγ, apte à induire une distribution de ligand(s) dans le milieu biologique cible, à une concentration efficace, c'est-à-dire apte à induire et/ou à amplifier l'expression des MMR d'au moins une partie des macrophages présents dans ce milieu -par exemple, un tissu ou une composition de sang périphérique-. Cette concentration est, en général, au moins équivalente à l'EC50 du(des) ligand(s) utilisé(s). Le milieu biologique contenant des macrophages selon l'invention correspond, dans ce cas particulier, à un milieu naturel intracorporel.

Le tableau 1 ci-après présente les valeurs EC50 pour certains ligands agonistes synthétiques de PPARγ.

Tableau 1: Activité transactivatrice des agonistes de PPARγ (CERIN - N°70, Mars/Avril 2002)

| Composés | EC50 (M) | |
|---|---|---|
| | PPARγ murin | PPARγ humain |
| troglitazone | 0,78 | 0,55 |
| pioglitazone | 0,55 | 0,58 |
| rosiglitazone | 0,076 | 0,043 |
| KRP-297 | 0,14 | 0,083 |
| JTT-501β | 0,089 | 0,083 |
| SB 213068 | 0,10 | 0,066 |
| GI 262570 | 0,00035 | 0,00034 |
| GW 1929 | 0,013 | 0,0062 |
| GW 7845 | 0,0012 | 0,00071 |
| GW 0207 | 0,14 | 0,044 |
| L-796449 | 0,010 | 0,0052 |

Selon le site visé, l'administration peut se faire par voie orale, rectale, ou parentérale (sous-cutanée, transdermique, intraveineuse, intramusculaire). Par ailleurs, selon le site visé, la distribution du composé actif peut circuler jusqu'à la cible via le système sanguin périphérique, ou l'atteindre par une administration topique (administration du composé directement au contact du site cellulaire à traiter). La forme galénique et les doses à administrer seront choisies en conséquence.

Compte tenu de la valeur EC50 de certains des ligands agonistes de PPARγ actuellement connus, on peut envisager, en particulier pour les composés cités précédemment, une utilisation à des doses efficaces sans risque de toxicité (par exemple, on peut prescrire la rosiglitazone à des doses journalières de l'ordre 4 à 8 mg pour un adulte de 70 kg). A noter que la plupart des ligands agonistes de PPARγ, en particulier ceux précédemment cités, ont déjà passé avec succès les tests de toxicité dans le cadre de l'obtention de l'autorisation de mise sur le marché, comme anti-inflammatoires ou hypolipidémiants.

Selon une variante de réalisation, conforme à l'invention et entrant également dans le cadre de la stimulation du système immunitaire par induction de l'expression du MMR chez un individu -en particulier immunodéprimé-, le milieu biologique comprenant des macrophages à stimuler peut être également extracorporel. Les macrophages sont alors préparés à partir d'un prélèvement sanguin d'un patient, puis sont traités avec une quantité efficace d'au moins un ligand agoniste de PPARγ, avant d'être réinjectés à ce même patient.

Eventuellement, les macrophages peuvent aussi provenir d'un donneur immunocompatible avec le patient receveur. En particulier, ce dernier est atteint de déficits immunitaires, notamment un déficit en macrophages matures.

Selon cette même perspective de stimulation de l'expression du MMR, l'invention s'étend à un procédé de traitement d'un tissu vivant ou d'une composition de sang périphérique, non isolés, d'un primate vivant -notamment de l'homme-, dans lequel on administre dans un tissu ou dans une composition de sang périphérique dudit primate, une quantité efficace d'au moins un ligand agoniste de PPARγ, adaptée pour induire une distribution de ligand(s) dans ledit tissu ou ladite composition de sang périphérique, à une concentration thérapeutiquement efficace, c'est-à-dire apte à induire et/ou à amplifier l'expression des MMR des macrophages présents dans ledit tissu ou ladite composition de sang périphérique.

Selon un autre aspect, l'invention a trait à un procédé d'élimination, intracorporel ou extracorporel, de micro-organismes d'origine bactérienne, fongique ou protozoaire, ayant, sur la face extérieure de leurs parois, des polysaccharides et/ou lipopolysaccharides avec des extrémités libres mannosylés et/ou fucosylés. Selon l'invention, qu'on place, dans un milieu infecté par lesdits micro-organismes et au contact de macrophages, une quantité efficace d'au moins un ligand agoniste de PPARγ à titre de facteur d'induction et/ou d'amplification de l'expression des récepteurs mannose des macrophages.

Avantageusement et selon l'invention, lesdits micro-organismes sont choisis parmi : *Streptococcus pneumoniae, Klebsellia pneumoniae, Cryptococcus neoformans, Mycobacterium tuberculosis, Candida albicans, Leishmania donovani, Pneumocystis carinii, Trypanosoma cruzi, Aspergillus fumigatus, pseudomonas aeruginosas, Legionella pneumophilla.*

Avantageusement et selon l'invention, au moins un des ligands agonistes de PPARγ est choisi parmi le groupe des ligands naturels : la 15d-PGJ$_2$, les prostaglandines A1 et D2, les acides 9- et 13-hydoxyoctadécanoïques, les dérivés des LDL oxydées.

Ce ligand peut également être choisi parmi le groupe des ligands synthétiques : la troglitazone, la rosiglitazone, la pioglitazone, la ciglitazone, l'englitazone, le GW 347845, le KRP-297, le JTT-501β, le SB 213068, le GI 262570, le GW 1929, le GW 7845, le GW 0207, le L-796449, l'indométhacine et l'ibuprofène.

Selon l'invention, l'utilisation d'un ligand agoniste de PPARγ pour éliminer un micro-organisme infectieux via des macrophages stimulés, peut intéresser un milieu artificiel infecté, par exemple un milieu de culture, ou un milieu naturel infecté, par exemple un tissu vivant ou le sang périphérique, isolé ou non d'un organisme pluricellulaire -notamment un primate, notamment vivant-.

L'invention s'étend en conséquence également à un procédé de fabrication d'une composition thérapeutique comprenant au moins un ligand agoniste de PPARγ à titre de composé actif apte à induire et/ou à amplifier l'expression des récepteurs mannose des macrophages.

Plus particulièrement, il s'agit d'un procédé de fabrication d'une composition thérapeutique -notamment une composition immunostimulante-destinée au traitement préventif ou curatif d'une pathologie appartenant au groupe des pathologies induites par des micro-organismes, d'origine bactérienne, fongique ou protozoaire qui ont, sur la face extérieure de leurs parois, des motifs mannosylés et/ou fucosylés, à l'exception de *Mycobacterium avium*.

Selon l'invention, on utilise une quantité thérapeutiquement efficace d'au moins un ligand agoniste du récepteur nucléaire PPARγ à titre de facteur d'induction et/ou d'amplification de l'expression des MMR.

Avantageusement et selon l'invention, il s'agit de micro-organismes choisis parmi : *Streptococcus pneumoniae, Klebsellia pneumonie, Cryptococcus neoformans, Mycobacterium tuberculosis, Candida albicans, Leishmania donovani, Pneumocystis carinii, Trypanosoma cruzi, Aspergillus fumigatus, Pseudomonas aeruginosas, Legionella pneumophilla.*

Avantageusement et selon l'invention, on utilise pour la fabrication de ladite composition thérapeutique au moins un ligand agoniste de PPARγ choisi parmi le groupe des ligands naturels : la 15d-PGJ$_2$, les prostaglandines A1 et D2, les acides 9- et 13-hydoxyoctadécanoïques, les dérivés des LDL oxydées.

Pour la fabrication d'une composition thérapeutique conforme à l'invention, on peut également choisir ce ligand parmi le groupe des ligands synthétiques : la troglitazone, la rosiglitazone, la pioglitazone, la ciglitazone, l'englitazone, le GW 347845, 1e KRP-297, le JTT-501β, le SB 213068, le GI 262570, le GW 1929, le GW 7845, le GW 0207, le L-796449, l'indométhacine et l'ibuprofène.

Une composition thérapeutique préparée conformément à un procédé selon l'invention, est ainsi adaptée pour induire et/ou amplifier l'expression du MMR. Aussi, elle peut être considérée comme une composition immunostimulante, capable d'améliorer le niveau de vigilance du système immunitaire vis-à-vis de nombreux micro-organismes infectieux, de stimuler la réponse immunitaire innée et d'amplifier les fonctions effectrices des macrophages lorsque ces derniers sont placés en présence de ces micro-organismes. Aussi, avantageusement et selon l'invention, elle est destinée à la médication de sujets immunodéprimés.

Avantageusement, une composition thérapeutique obtenue par un procédé de fabrication conforme à l'invention, peut également comprendre un ou plusieurs autres principes actifs, notamment pour procurer un effet synergique. En particulier, outre un ligand agoniste de PPARγ, elle peut avantageusement renfermer une quantité d'extrait de paroi d'un micro-organisme vis-à-vis duquel on cherche à se défendre.

La forme galénique d'une composition thérapeutique conforme à l'invention est choisie en fonction de la voie d'administration et peut être réalisée par les techniques traditionnelles.

L'invention s'étend également à un traitement curatif de pathologies susceptibles d'être induites par des micro-organismes, d'origine bactérienne, fongique ou protozoaire, à motifs mannosylés et/ou fucosylés. Il s'agit notamment de micro-organismes tels que : *Streptococcus pneumoniae, Klebsellia pneumoniae, Cryptococcus neoformans, Mycobacterium tuberculosis, Candida albicans, Leishmania donovani, Pneumocystis carinii, Trypanosoma cruzi, Aspergillus fumigatus, Pseudomonas aeruginosas, Legionella pneumophilla.*

L'invention s'adresse aux personnes susceptibles d'être exposées à ces agents infectieux, ou ayant été infectées par ces derniers. Elle s'adresse, en particulier et de préférence, aux patients atteints de déficits immunitaires, innés ou acquis, et qui de ce fait sont particulièrement sensibles aux maladies dites opportunistes ; des maladies qui sont essentiellement causées par des pathogènes fongiques (par exemple, des candidoses), connus pour présenter, à leur surface, un grand nombre de motifs mannosylés et/ou fucosylés.

Dans un procédé selon l'invention, l'élimination du pathogène implique essentiellement une reconnaissance du pathogène médiée par les récepteurs mannose des macrophages dont l'expression a été induite et/ou amplifiée par un ligand agoniste de PPARγ, naturel ou synthétique. Dans un procédé selon l'invention, on met avantageusement en oeuvre un procédé de stimulation de l'expression du MMR comme décrit précédemment.

En particulier, on traite le patient en lui administrant une quantité, efficace et adaptée, d'au moins un ligand agoniste de PPARγ. Cette quantité est, en général, au moins équivalente à l'EC50 du(des) ligand(s) utilisé(s). Avantageusement, on utilise une composition thérapeutique dont le procédé de fabrication a été ci-dessus présenté.

L'invention concerne aussi un procédé de stimulation de macrophages, un procédé d'élimination de pathogènes à motifs mannosylés et/ou fucosylés, un procédé de fabrication d'une composition thérapeutique, caractérisés, en combinaison, par tout ou partie des caractéristiques ci-dessus ou ci-après.

D'autres buts, caractéristiques et avantages de l'invention apparaîtront à la lecture des essais expérimentaux suivants qui se réfèrent aux figures annexées, dans lesquelles :

- la figure 1 est une représentation graphique de l'effet d'inhibition des ligands agonistes de PPARγ sur la prolifération de *C. albicans,*
- la figure 2 est une représentation graphique de la capacité des macrophages à phagocyter *C. albicans* selon que PPARγ soit ou non inhibé,
- la figure 3 montre des spectres de distribution de fluorescence reflétant l'induction du récepteur mannose par divers ligands agonistes de PPARγ,
- la figure 4 montre des spectres de distribution de fluorescence reflétant l'implication de PPARγ dans la régulation de l'expression du MMR,
- la figure 5 est une représentation graphique de l'implication de PPARγ dans la régulation de l'expression du MMR,
- la figure 6 est une représentation graphique de l'effet d'amplification de divers ligands agonistes de PPARγ sur l'expression du MMR,
- la figure 7 est une représentation graphique de l'effet *in vivo* de la 15d-PGJ$_2$ sur la capacité des macrophages à produire des agents oxydants,
- la figure 8 est une représentation graphique du niveau d'expression des MMR chez des souris SWISS et RAG-2 infectées par *C. albicans,*
- la figure 9 est une représentation graphique de l'effet des ligands agonistes de PPARγ sur l'expression du MMRde souris homozygotes (+/+) et hétérozygotes (-/-) pour le gène de PPARγ,
- la figure 10 est une représentation graphique de l'effet *in vivo* des ligands agonistes de PPARγ sur la prolifération de *C. albicans,* au niveau du tractus gastrointestinal par *C. albicans.*

• EXPERIMENTATIONS *IN VITRO*

Les travaux menés *in vitro* ont permis de mettre en évidence une stimulation des propriétés anti-infectieuses des macrophages vis-à-vis de différents micro-organismes présentant à leur surface des motifs mannosylés -notamment *C. albicans-,* via l'augmentation de l'activité phagocytaire des macrophages et de leur capacité à produire des agents oxydants.

Il a été également montré que le récepteur associé à l'augmentation de la phagocytose de *C. albicans* par les macrophages traités par les ligands spécifiques de PPARγ était bien le MMR. De plus, l'utilisation d'un antisens spécifique du récepteur mannose a permis de montrer une implication directe de l'induction de l'expression de ce récepteur dans l'augmentation du métabolisme oxydatif des macrophages. Enfin, des expériences de transfection d'un vecteur rapporteur CMV-PPARγ dans des cellules RAW 264.7, connues pour exprimer faiblement PPARγ, ont révélé une régulation de l'expression des MMR par une voie de signalisation particulière dans laquelle est impliqué le récepteur nucléaire PPARγ.

Le matériel et les méthodes utilisés sont présentés ci-après.

Les souches de *C. albicans* ont été isolées à partir d'échantillons de sang d'un patient atteint de candidose, selon des techniques classiques.

Les macrophages, quant à eux, proviennent soit du péritoine de souris femelles SWISS (Centre d'élevage, Etablissement Janvier, France), soit du sang périphérique de donneurs sains. Ces macrophages, de souris ou d'humain, sont cultivés dans un milieu de culture SFM ("*serum-free medium*") optimisé pour la culture des macrophages, commercialisé par la société GibcoBRL, France. L'adhésion des cellules se produit après environ 2 heures de culture à 37°C sous atmosphère à 5 % de CO$_2$. Les cellules n'adhérant pas à la boîte de culture sont éliminées par rinçage au PBS (GibcoBRL, France).

Pour les différents tests, les cellules adhérentes restant sur les boîtes sont stimulées par la 15d-PGJ$_2$, la ciglitazone ou la rosiglitazone (MERCK-LIPHA, France) à différentes concentrations. Pour étudier la prolifération de *C.*

*albicans,* des mesures d'incorporation de [3H]uracile (AMERSHAM PHARMACIA BIOTECH, France) dans les ARN sont effectuées avec des cellules en cours de multiplication.

Pour chaque essai, $2.10^5$ macrophages par puits sont cultivés pendant 2 heures sur des plaques Falcon® de 96 puits, dans du SFM à 37°C et sous atmosphère à 5 % de $CO_2$. Les macrophages sont stimulés par les agonistes de PPARγ pendant 24 heures, puis infectés par des blastoconidies de *C. albicans* dans une proportion macrophages/ levures de 1:200, pendant 6 heures à 37°C, sous atmosphère humidifiée contenant 5 % de $CO_2$. Pour certains tests, l'uracile tritié (1 μCi) est ajouté à chaque puits.

Après un challenge de 6 heures, les surnageants des puits contenant les blastospores non-adhérents sont collectés et centrifugés. Le culot est lavé au PBS. La radioactivité incorporée dans les ARN de levure est comptée avec le fluide de scintillation BCS (AMERSHAM PHARMACIA BIOTECH, France). Les monocouches contenant des levures adhérentes ou phagocytées sont lavées deux fois au PBS, puis lysées avec NaOH (1M) et neutralisées par HCl (1M). Le liquide de scintillation est alors ajouté à chaque fiole et la radioactivité est comptée au moyen d'un compteur Pharmacia LKB1217. Le nombre de désintégrations par minute (dpm) permet d'apprécier le niveau de prolifération de *C. albicans* pour chacun des essais.

Pour étudier le mécanisme d'ingestion de *C. albicans* par les macrophages, des levures préalablement marquées par [3H]uracile sont utilisées. Trois colonies de *C. albicans* sont dispersées dans 1 ml de bouillon Sabouraud contenant 150 μCi de [3H]uracile. Après 24 heures de culture à 37°C sous agitation, les blastospores marqués sont centrifugés et resuspendus dans le SFM enrichi avec 1,8 % de glucose. Pour chaque essai, les monocouches de macrophages cultivées sur les plaques 24 puits sont traitées avec les agonistes de PPARγ pendant 20 heures, puis infectées avec *C. albicans* marqué et viable (dans une proportion macrophages/levures de 1:1) et laissées à incuber pendant 1 heure à 37°C, puis 2 heures à 5°C. Soumis à 5°C, les mécanismes de phagocytose sont bloqués. Au terme de chacune des périodes d'incubation (1 heure ou 2 heures), le milieu de culture est prélevé, les monocouches de macrophages sont rincées deux fois au PBS. Les solutions de rinçage sont conservées. Les monocouches sont lysées par NaOH (1M) puis neutralisées par HCl (1M). La radioactivité du surnageant et celle des solutions de rinçage, ainsi que la radioactivité contenue dans le lysat cellulaire sont comptées séparément avec le fluide de scintillation BCS au moyen d'un compteur de scintillation de liquide. L'index de phagocytose est évalué de la manière suivante :

$$\% \text{ Phagocytose} = \frac{\Delta_{(37°C - 5°C)}\text{dpm}_{\text{monocouches}}}{\Delta_{(35°C - 5°C)}\text{dpm}_{\text{monocouches}} + \text{dpm}_{\text{surnageant}}}$$

Pour les essais de cytométrie en flux, les macrophages sont rincés au PBS glacé. Le marquage des MMR est réalisé pendant une heure, dans la glace, à la manière d'un ligand spécifique conjugué avec de l'albumine bovine mannosylée marquée à la fluorescéine FITC (840 nM) (SIGMA, France). Afin de faciliter la sélection des populations de cellules viables, une coloration à l'iodure de propidium est réalisée. Toutes les analyses sont effectuées par le FACScalibur (Becton-Dickinson) en utilisant le logiciel CellQuest.

Les analyses de l'ADNc des MMR par RT-PCR sont menées de la manière suivante. Les ARN totaux sont isolés des cellules au moyen du réactif "Extract All" (EUROBIO, France). Les ARN sont reverse-transcrits en utilisant le kit "First-Strand DNA Synthesis" (PROMEGA, France), durant 30 cycles d'amplification (30 sec. à 95°C, 1 min à 48°C et 1 min à 68°C). Les séquences des amorces (MMR sens et MMR antisens) utilisées pour obtenir l'ADNc du MMR sont présentées dans le tableau 2.

| | | Noms | Séquences |
|---|---|---|---|
| Amorces | PCR | MMR sens | 5'GCTCTAGAATGGAACACACACTCTGGGCCATG3' |
| | | MMR antisens | 5'TACCACTTGTTTTCAAACTTGAA3' |
| Antisens | ARNm | ODN | 5'GAGTGGGCTTACGTGGTTGT3' |
| | | ANS | 5'GGTTCCTTTCCTGATTTACG3' |

Tableau 2

Pour les tests de transfection, les macrophages de la lignée cellulaire murine RAW 267.4 (QBiogene, France) sont maintenus en phase de croissance exponentielle dans du DMEM complémenté avec 10 % de PBS. La veille de la transfection, les cellules sont transférées sur des plaques de 6 puits. Le milieu est alors remplacé par du DMEM dépourvu de sérum et les cellules sont transfectées de manière transitoire pendant 18 heures à 60-80 % de confluence avec le réactif de transfection FuGENE 6 (ROCHE, Suisse) contenant le vecteur à transfecter (CMV-luciférase ou CMV-PPARγ), en proportion ADN/FuGENE 6 de 1:2, avec une quantité d'ADN de 3 µg. Le jour de la stimulation, le milieu est remplacé par le milieu complet (DMEM + FBS 10 %) contenant les différentes molécules à tester. Les cellules sont collectées 24 heures plus tard puis analysées par cytométrie en flux. La transfection du vecteur CMV-luciférase (PROMEGA, France) sert de contrôle. La séquence pCMX-mPPARγ (Forman B.M. et al., Cell, 1995, 83:803-812) code pour le récepteur nucléaire PPARγ murin.

Concernant le traitement à l'antisens ODN, une séquence complémentaire à la région 3' non traduite de l'ARNm du MMR (Yamamoto et al., Infect Immun, 1997, 65:1077-82), on incube l'antisens ODN (100 nM) avec 10 µg de lipide cationique Lipofectin (GIBCO, France) à 37°C pendant 5 min selon la méthode Capacciolis et al. (Biochem Biophys Res Commun, 1998, 197:818-25) puis on l'ajoute aux macrophages cultivés dans le DMEM sans sérum. Les cellules sont incubées pendant 4 heures à 37°C avec 5 % de $CO_2$ atmosphérique. Le milieu est remplacé par du SFM contenant l'antisens ODN mais sans Lipofectin. Les cultures sont incubées pendant 3 jours avec un changement journalier du milieu. La concentration de l'antisens ODN utilisée n'entraîne aucune toxicité significative sur les macrophages. Les macrophages non traités et les macrophages traités avec la Lipofectin seule servent de contrôle. La séquence nucléique de l'antisens ODN est exposée dans le tableau 2. Le même traitement est réalisé avec un antisens non spécifique de l'ARNm du MMR, l'antisens ANS dont la séquence nucléique est exposée dans le tableau 2.

Pour les tests de production d'agents oxydants, l'explosion respiratoire oxygène-dépendante des macrophages traités par les agonistes de PPARγ est mesurée par chimiluminescence. Les macrophages ($2.10^5$) sont transférés sur des boîtes de culture de 5 mm de diamètre. Après activation des cellules, la production d'agents oxydants par les macrophages, en présence ou en absence de *C. albicans*, est mesurée par chimiluminescence en présence de luminol à 37°C. L'émission de chimiluminescence est enregistrée en continu pendant 1 heure après incubation des cellules avec le luminol (66 µM). Pour évaluer la production de l'anion superoxyde ($O_2^-$), la chimiluminescence est mesurée en présence de SOD, un inhibiteur spécifique de la production d'anion superoxyde. Pour évaluer la production d'oxyde nitrique (NO), la chimiluminescence est mesurée en présence de la L-NMMA (NG-monométhyl-arginine, un compétiteur spécifique de la L-arginine pour la production de NO). Aucun des inhibiteurs n'affecte la viabilité aux concentrations utilisées.

Les analyses statistiques se basent sur la mesure de la surface sous la courbe et sont exprimées en coups x secondes.

Essai 1 : Stimulation de l'activité candistatique des macrophages péritonéaux murins par les ligands spécifiques des PPARγ.

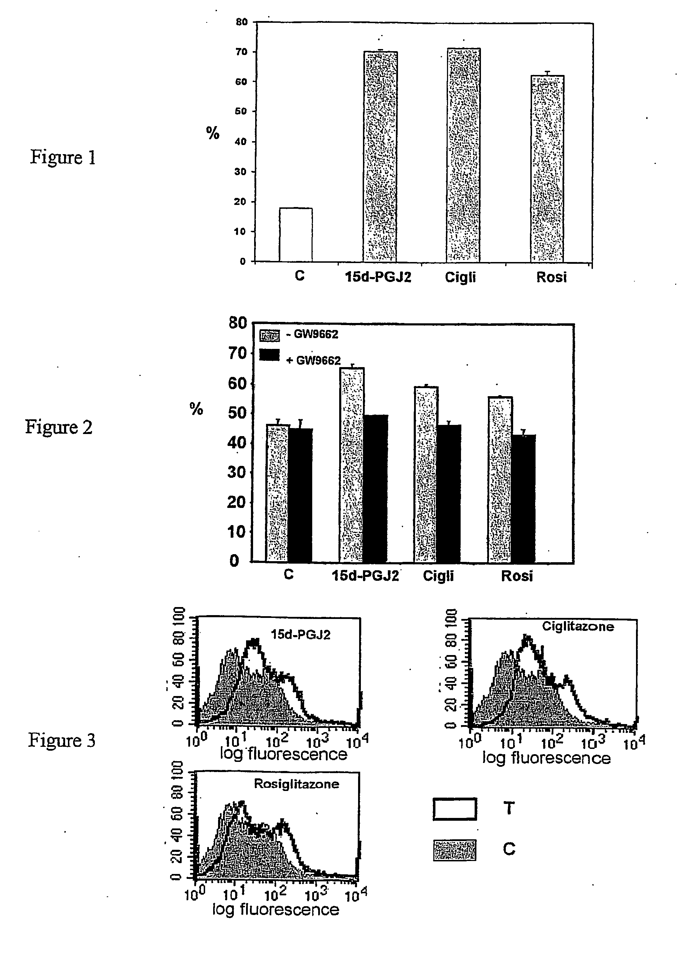

L'action des macrophages traités par les ligands spécifiques de PPARγ dans l'inhibition de prolifération de *C. albicans* est étudiée face à un challenge *in vitro* de 6 heures avec la souche 98-26135 (Coste A. et al., J. Antimicrobial Chemotherapy, 2002, 49:731-740). La prolifération de *C. albicans*, a été déterminée en présence de macrophages cultivés selon la technique radiométrique. La figure 1 expose l'inhibition de la prolifération (en %) de *C. albicans* en présence de macrophages non traités (C) ou traités (T) par la 15d-PGJ₂, la ciglitazone (Cigli), et la rosiglitazone (Rosi).

La figure 1, montre que les macrophages développent une activité fongistatique propre, inhibant de 18 % la croissance de *C. albicans*. En présence des ligands agonistes de PPARγ dans le milieu, les macrophages possèdent une activité candistatique plus importante. En effet, l'inhibition de prolifération est multipliée environ par trois (par rapport au contrôle) quand les macrophages sont traités par-les différentes drogues. Les ligands agonistes de PPARγ stimulent l'activité candistatique des macrophages.

Essai 2 : Augmentation de la phagocytose de *C. albicans* et de la production d'agents oxydants par les ligands spécifiques des PPARγ.

Comme les mécanismes de défense contre *C. albicans* sont essentiellement dépendants de la capacité des macrophages à phagocyter les levures et à exercer leur activité fongicide en produisant des molécules toxiques (IROs et NO), la modulation de la phagocytose de cette levure ainsi que la production d'agents oxydants des macrophages traités par les ligands spécifiques de PPARγ, en absence ou présence du GW 9662 (CAYMAN CHEMICAL, Michigan, USA), un antagoniste irréversible de PPARγ, ont été étudiées.

La figure 2 fait état de la capacité de phagocytose (exprimée en index de phagocytose) des macrophages péritonéaux non traités (C) ou traités par des ligands spécifiques de PPARγ (15d-PGJ₂, ciglitazone et rosiglitazone) en absence ou présence du GW 9662, face à un challenge d'une heure avec *C. albicans* dans les conditions d'un rapport macrophages/levures de 1:1.

La figure 2 montre qu'il existe une augmentation significative du nombre de levures phagocytées quand les macrophages sont traités par les ligands agonistes de PPARγ. Le nombre de levures internalisées par les macrophages est augmenté de 19 % par le traitement avec la 15d-PGJ₂, de 13 % avec la ciglitazone (Cigli), de 10 % avec la rosiglitazone (Rosi), comparé aux macrophages non traités (C) qui ont un index de phagocytose de 46 %.

De plus, l'augmentation de la capacité de phagocytose des macrophages traités par les agonistes de PPARγ est inhibée par le GW 9662.

La capacité des macrophages à produire de l'anion superoxyde et du monoxyde d'azote et leur modulation par la 15d-PGJ$_2$ ont été également étudiées par la méthode de chimiluminescence qui permet d'évaluer non seulement la production d'anions superoxydes mais aussi celle du peroxynitrite produit au cours de l'interaction entre le monoxyde d'azote et l'anion superoxyde.

Afin d'évaluer la production d'anion superoxyde, la chimiluminescence a été mesurée- en présence ou non de SOD (enzyme métabolisant O$_2^-$ en H$_2$O$_2$) alors que la production de monoxyde d'azote est évaluée en présence d'un compétiteur de l'arginine qui est la L-NMMA.

Tableau 3 : Effet des ligands agonistes de PPARγ sur la production d'agents oxydants par les macrophages en présence ou en absence de levure. L'évaluation de la production d'agents oxydants a été déterminée sur des macrophages en culture par la technique de chimiluminescence.

| Production d'agents oxydants pendant 1 heure (C x S : coups x secondes) | | | | |
|---|---|---|---|---|
| Traitements | - Levure | + Levures | | |
| Contrôle | 7874 ± 65 | 7664 ± 180 | SOD | L-NMMA |
| 15D-PGJ$_2$ | 7469 ± 238 | 13905 ± 328 | 7228 ± 212 | 14258 ± 216 |

Le tableau 3 montre que les macrophages, mis en présence des différents traitements, produisent la même quantité basale d'agents oxydants que les macrophages résidents. Les ligands spécifiques de PPARγ seuls (en l'absence de *C. albicans*) n'induisent pas d'augmentation de la production des agents oxydants par les macrophages.

Par contre, l'ajout des levures augmente la production d'agents oxydants. Les ligands agonistes de PPARγ pré-activent la capacité des macrophages à produire les agents oxydants. En réponse à une infection par *C. albicans*, les macrophages dans cet état d'activation produisent principalement de l'anion superoxyde. En effet, en présence de SOD, un inhibiteur spécifique de la production des superoxydes, la production d'agents oxydants est significativement inhibée. A l'inverse, la chimiluminescence mesurée en présence de la L-NMMA n'est pas inhibée. Ainsi, les macrophages dans cet état d'activation ne produisent pas du monoxyde d'azote.

Ces résultats qui montrent que l'antagoniste de PPARγ bloque les effets candistatiques des ligands de PPARγ suggèrent que PPARγ est impliqué dans l'augmentation de l'activité fongicide des macrophages sous l'effet des différents traitements.

Essai 3 : Induction de l'expression des récepteurs mannose par les ligands spécifiques de PPARγ.

Comme le récepteur mannose est impliqué dans la phagocytose *C. albicans* ainsi que de nombreux autres micro-organismes pathogènes présentant sur la face extérieure de leurs parois des résidus mannosylés, l'expression de ce récepteur à la surface des macrophages traités par les ligands agonistes de PPARγ a été étudiée par cytométrie en flux grâce à la fixation de molécules d'albumine bovine mannosylée (mBSA) marquées à la fluorescéine (FITC, fluorothioisocyanate).

Les macrophages ont été cultivés pendant 24 heures en présence de 1 μM de 15d-PGJ$_2$, de 5 μM de ciglitazone ou de 5 μM de rosiglitazone. Les cellules sont ensuite incubées dans la mBSA marquée au FITC et analysées par cytométrie en flux. La distribution de la fluorescence de la population cellulaire analysée est représentée à la figure 3.

La figure 3 montre que la quantité d'albumine bovine mannosylée marquée à la fluorescéine présente sur les macrophages est significativement augmentée quand les cellules sont traitées (T) par les ligands spécifiques de PPARγ en comparaison avec les cellules non traitées (C). La spécificité de la mBSA pour le récepteur mannose suggère que les ligands spécifiques de PPARγ induisent l'expression des récepteurs mannose à la surface des macrophages.

L'analyse de régression des résultats exposés aux figures 2 et 3 indique une association linéaire entre la quantité de MMR et la capacité de phagocytose après traitement par les différents ligands de PPARγ des macrophages, démontrant ainsi une vraie corrélation entre l'expression des récepteurs mannose influencée par les différents traitements et la capacité de ces cellules à internaliser *C. albicans* (r = 0,942).

Essai 4 : Implication directe de PPARγ dans la régulation de l'expression des récepteurs mannose par les ligands spécifiques des PPARγ.

Afin de déterminer si le facteur de transcription PPARγ est impliqué dans la sur-expression du récepteur mannose induite par les ligands spécifiques de PPARγ, deux approches différentes ont été utilisées. Tout d'abord, nous avons transfecté (pendant 18 heures) deux vecteurs rapporteurs, CMV-luciférase et CMV-PPARγ, où le gène de la luciférase et le gène de PPARγ sont sous la dépendance d'un promoteur fort, celui du cytomégalovirus. Nous avons effectué ces transfections dans la lignée RAW 264.7, connue pour exprimer faiblement PPARγ. Après traitement avec les ligands spécifiques de PPARγ les récepteurs mannose présents à la surface des cellules ont été quantifiés par cytométrie en flux.

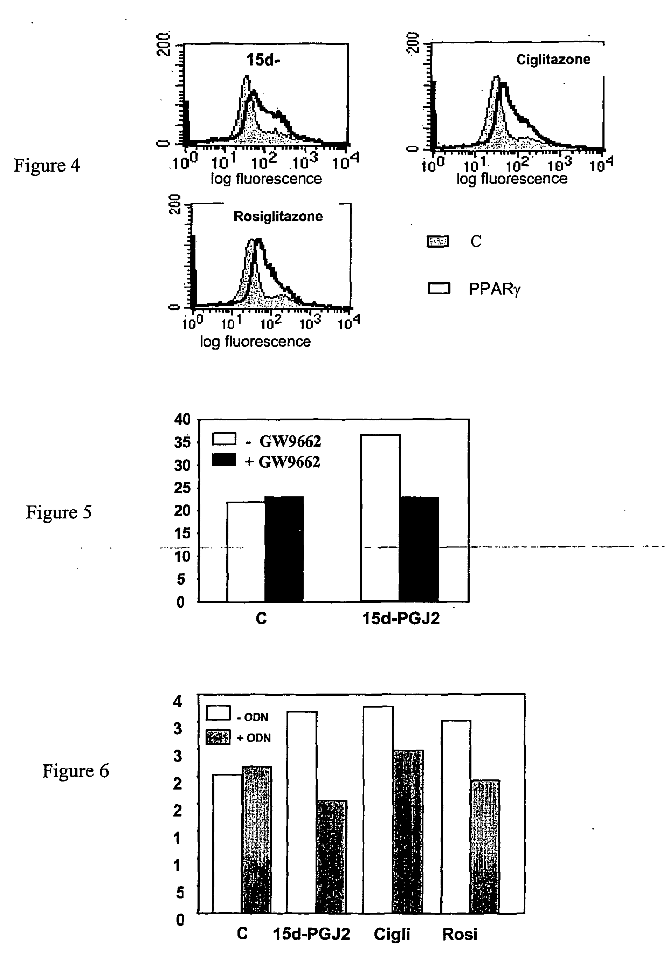

Les cellules RAW 264.7 ont ensuite été cultivées pendant 24 heures en présence d'1 μM de 15d-PGJ$_2$, de 5 μM de ciglitazone ou de 5 μM de rosiglitazone. Les cellules sont incubées avec la mBSA marquée au FITC et analysées par cytométrie en flux. La distribution de la fluorescence de la population cellulaire analysée est représentée à la figure 4 et la moyenne géométrique des intensités de fluorescence correspondantes, reportées dans le tableau 4 ci-après.

Tableau 4

| Traitements | Moyenne géométrique de l'intensité de fluorescence | |
|---|---|---|
| | CMV-LUC | CMV-PPAR-γ |
| Contrôle | 47,51 | 53,87 |
| 15d-PGJ$_2$ | 44,29 | 73 |
| Ciglitazone | 38,51 | 64,3 |
| Rosiglitazone | 37,94 | 61,51 |

Dans une deuxième étape, l'induction de l'expression du récepteur mannose des macrophages péritonéaux murins sous l'effet des différents traitements a été étudiée par cytométrie de flux en présence d'un antagoniste irréversible de PPARγ, le GW 9662.

La figure 4 montre que dans les cellules RAW 264.7 contrôle (C), cellules transfectées avec CMV-luciférase, le traitement avec les ligands de PPARγ n'a aucun effet sur l'expression des récepteurs mannose. Par ailleurs, la transfection des cellules RAW 264.7 avec le vecteur CMV-PPARγ restaure l'effet des différents traitements sur l'induction de l'expression des récepteurs mannose. Ceci démontre que PPARγ est directement impliqué dans la régulation de l'expression du récepteur mannose par les ligands spécifiques de PPARγ.

Ces résultats sont confirmés par la figure 5 qui montre que l'augmentation de l'expression du récepteur mannose à la surface des macrophages sous l'effet de la 15d-PGJ$_2$ est antagonisée par le GW 9662. Pour ce faire, les macrophages ont été cultivés 24 heures en présence ou en absence du GW 9962 (0,1 μM), puis incubées avec la mBSA marquée au FITC et analysées par cytométrie en flux.

Ces deux expériences montrent l'implication de PPARγ dans la sur-expression des récepteurs mannose à la surface des macrophages.

Essai 5 : L'induction de l'expression du récepteur mannose à la surface des macrophages sous l'effet de différents agonistes de PPARγ est dépendante d'une régulation transcriptionnelle.

Afin de déterminer si l'activation de PPARγ conduit à une induction transcriptionnelle du récepteur mannose deux techniques différentes ont été réalisées en parallèle. Dans cet objectif, une première approche a consisté à transfecter un antisens spécifique de l'ARNm des récepteurs mannose dans des macrophages et les récepteurs mannose présents à la surface des cellules ont été quantifiés par cytométrie en flux. Parallèlement, une autre technique a été utilisée et a consisté à quantifier directement l'expression des ARNm du récepteur mannose, sous l'effet des différents traitements, par RT-PCR quantitative en temps réel.

Pour ce faire, les macrophages ont été traités pendant 3 jours avec l'antisens spécifique de l'ARNm du récepteur mannose (ODN). Les macrophages sont cultivés 24 heures en présence d'1 μM de 15d-PGJ$_2$, 5 μM de ciglitazone, ou 5 μM de rosiglitazone. Les cellules ont été ensuite incubées avec la mBSA marquée au FITC, puis analysées par cytométrie en flux. L'intensité de la fluorescence analysée est représentée à la figure 6.

La figure 6 montre qu'en présence de l'antisens, la quantité de fluorescence est significativement inhibée quel que soient les traitements utilisés. Ces résultats indiquent d'une part que l'augmentation de la quantité de mBSA fixée sur les macrophages résulte bien d'une induction de l'expression des récepteurs mannose et, d'autre part, que cette sur-expression est le reflet direct d'une augmentation de l'expression des ARNm du récepteur mannose.

Essai 6 : Le récepteur mannose est impliqué dans la modulation des fonctions oxydatives des macrophages par les ligands de PPARγ.

Cette étude a été réalisée afin de déterminer si l'augmentation de la production d'agents oxydants sous l'effet des agonistes de PPARγ dépendait ou non de la modulation des récepteurs mannose par ces différents traitements. Dans cet objectif, la modulation de la production d'agents oxydants par les macrophages transfectés par l'antisens spécifique des ARNm des récepteurs mannose a été étudiée. Après traitement avec les agonistes de PPARγ, le dosage de la quantité d'agents oxydants produits par les macrophages transfectés a été réalisé par chimiluminescence. Les résultats sont présentés dans le tableau 5.

Tableau 5 : Implication du récepteur mannose dans la production d'agents oxydants des macrophages traités par les ligands de PPARγ. La production d'agents oxydants a été déterminée sur des macrophages en culture par la technique de chimiluminescence. C x S : coups x secondes pendant 1h. Les macrophages sont incubés pendant 3 jours avec un antisens spécifique (ODN) ou avec un antisens non spécifique (ANS) de l'ARNm du récepteur mannose.

| Production d'agents oxydants (coups x secondes) | | | |
|---|---|---|---|
| Traitements | sans antisens | avec ODN | avec ANS |
| Contrôle | $6737 \pm 139$ | $6795 \pm 178$ | $6972 \pm 90$ |
| 15d-PGJ$_2$ | $9161 \pm 85$ | $7012 \pm 98$ | $9678 \pm 106$ |
| ciglitazone | $8398 \pm 104$ | $7109 \pm 155$ | $8322 \pm 183$ |
| rosiglitazone | $8960 \pm 179$ | $7197 \pm 77$ | $8813 \pm 190$ |

Le tableau 4 montre que l'augmentation de la production d'agents oxydants par les macrophages induite par les traitements est antagonisée par l'antisens spécifique de l'ARNm du récepteur mannose Avec l'antisens non spécifique du récepteur mannose (ANS) aucun effet sur l'inhibition de la production d'agents oxydants n'est observé. Ainsi, la production de radicaux libres oxygénés est corrélée avec l'induction de l'expression des récepteurs mannose.

Essai 7 : Effet des ligands de PPARγ (rosiglitazone, ciglitazone, 15d-PGJ$_2$) sur l'expression des récepteurs mannose de monocytes humains.

L'étude a été réalisée sur des monocytes humains en culture, préparés à partir de cellules mononuclées de sang périphérique de donneurs sains.

La séparation des cellules mononuclées a été réalisée à partir de poches de résidus de cytophérèse, par une méthode standard de gradient de Ficoll-Hypaque (Bureau et al., J. Biol. Chem., 2001, 276:23077-23083). Les monocytes ont été isolés des cellules mononucléées, par adhésion après 2 heures de culture à 37°C et sous atmosphère à 5 % de CO$_2$ dans un milieu sans sérum pour macrophages (le SFM pour macrophage d'INVITOGEN CORP., France) et complémenté avec de la L-glutamine. Les cellules non adhérentes ont été éliminées par deux lavages avec une solution saline de Hanks. Les cellules adhérentes (85 % des monocytes/macrophages) ont été cultivées dans du SFM pour macrophage.

Il a pu être établi, sur ces cultures de monocytes, que les ligands agonistes de PPARγ induisent l'expression du récepteur mannose après 18 heures de culture.

De plus, il a été montré que la phagocytose de *C. albicans* est augmentée proportionnellement à l'induction des récepteurs mannose.

Enfin, il a été établi que les monocytes différenciés par les ligands agonistes de PPARγ tuent *C. albicans* par un mécanisme dépendant de la production d'anions superoxydes déclenché lors de l'interaction *C. albicans*-récepteur mannose.

• EXPERIMENTATIONS *IN VIVO*

Suite aux travaux menés obtenus *in vitro* qui montraient que le traitement des macrophages avec les ligands de PPARγ stimulait l'activité antifongique via PPARγ, des travaux ont été menés *in vivo* en vue de valider ces résultats.

L'étude *in vitro* a été réalisée sur des lots de 10 souris, soit immunocompétentes (SWISS) ou immunodéficientes (RAG-2), qui ont été infectées par *C. albicans*. Ces souris ont été traitées, ou non, par la 15d-PGJ$_2$ ou par la rosiglitazone.

Dans un premier temps, il a été vérifié que les différents traitements réalisés *in vivo* modifiaient bien la capacité de phagocytose des macrophages.

Dans un deuxième temps, l'expression du récepteur mannose à la surface des macrophages extraits des souris, traitées *in vivo* par la 15d-PGJ$_2$ ou par la rosiglitazone, a été étudiée par cytométrie en flux.

Les souris immunodéficientes type RAG-2 (Centre d'élevage du CNRS, Orléans, France) possédant un déficit combiné sévère de l'immunité à médiation cellulaire et humorale, n'ont pas de lymphocytes B et T, sont utilisées en pathologie virale type SIDA/HIV.

Dans ce modèle utilisant des RAG-2, il a été possible de reproduire une candidose orogastrointestinale identique à celle observée chez les patients immunodéprimés. Nous avons ainsi examiné, *in vivo,* l'efficacité antifongique de cette nouvelle stratégie thérapeutique. En parallèle, ce même protocole a été réalisé sur des souris SWISS immunocompétentes (Centre d'élevage - Etablissement Janvier, France).

Les souris hébergées dans des cages stériles ont été infectées par gavage de 500 μl d'une suspension de levures ($2.10^7$ cfu.ml$^{-1}$). Cette quantité de levure est suffisante pour établir des candidoses orogastrointestinales chez toutes les souris.

Des doses pharmacologiques de 15d-PGJ$_2$ (0,01 $\mu$moles) ou de rosiglitazone (0,1 $\mu$moles) ont été injectées par voie intra-péritonéale un jour avant l'infection par *C. albicans*. Ces injections ont été renouvelées tous les jours durant 5 jours.

Les macrophages ont été ensuite recueillis par lavage de la cavité péritonéale des souris infectées traitées, ou non, soit par la 15d-PGJ$_2$ soit par la rosiglitazone. La suspension macrophagique a été ajustée à 10$^6$/ml dans du SFM (GibcoBRL, France) complémenté de glutamine, de pénicilline et de streptomycine et a été distribuée à raison de 1 ml par puits dans les microplaques de 24 puits. Après une incubation de 2 heures à l'étuve à 37°C, 5 % CO$_2$, les cellules non adhérentes ont été éliminées par deux rinçages en PBS. A la suite de ces 2 heures, les tapis cellulaires en monocouches contiennent 95 % de macrophages et sont utilisés en vue d'évaluer leur capacité de phagocytose ainsi que leur capacité à produire des agents oxydants.

Afin d'évaluer la capacité des macrophages à phagocyter *C. albicans,* une technique basée sur l'incorporation de nucléotides radioactifs (Uracile tritié) au niveau de l'ARN des levures en voie de multiplication a été réalisée. Les levures pré-marquées sont déposées sur les tapis macrophagiques et mises à incuber à 37°C sous 5 % CO$_2$. A l'issue d'une heure d'incubation, la radioactivité des surnageants et des produits de rinçage des puits, ainsi que celle contenue dans les lysats cellulaires obtenus par lyse avec du NaOH (1N) seront mesurées indépendamment. Sachant que la radioactivité mesurée dans le lysat est proportionnelle à la quantité de levures phagocytées et que celle mesurée dans les surnageants est proportionnelle à la quantité de levures externes, le pourcentage de levures phagocytées par rapport à la quantité de levures totales sera déterminé selon la formule suivante :

$$\% \text{ Phagocytose} = \frac{\text{dpm}_{\text{lysat}}}{\text{dpm}_{\text{lysat}} + \text{dpm}_{\text{surnageant}}}$$

Afin d'évaluer la production d'agents oxydants (radicaux libres oxygénés et monoxyde d'azote) par les macrophages après la mise en culture des macrophages, les activités conjointes de la NADPH oxydase et de la NOsynthase inductible sont mesurées par chimiluminescence en présence d'une sonde chimiluminogène, Luminol (SIGMA, France) : 5-amino2,3-diydro-1,4-phtalazine-dione. La production de chimiluminescence sera analysée avec un luminomètre (Wallac Victor II, Finlande) piloté par un ordinateur. Le luminol permet, en présence d'agents oxydants, l'émission de photons captés par le photomultiplicateur de la chambre noire du luminomètre thermostaté à 37°C et transformés en impulsions électriques quantifiables. Les valeurs de chimiluminescence seront enregistrées continuellement pendant 1 heure après incubation des cellules avec le luminol (66 $\mu$m) et en présence ou non de levures.

Pour le MMR, les macrophages des souris infectées par *C. albicans* et traitées ou non *in vivo,* soit par la 15d-PGJ$_2$ soit par la rosiglitazone, ont été recueillis par lavage de la cavité péritonéale de ces souris. La suspension est ajustée à 10$^6$/ml dans du SFM et mise à incuber pendant 1 heure à 4°C avec de l'albumine bovine mannosylée (mBSA) marquée à la fluorescéine (14 $\mu$M). La quantité de molécule de mBSA fixée, reflet direct de la quantité de récepteurs mannose présente sur les cellules, a été analysée à l'aide d'un cytomètre en flux (FACScalibur, Becton Dickinson).

Par la suite, il a été établi que les traitements des souris par les ligands agonistes de PPAR$\gamma$ modifiaient l'infection candidosique au niveau de plusieurs organes (oesophage, estomac, caecum, reins, foie). Pour ce faire, après infection par *C. albicans,* les souris SWISS et RAG-2 ont été traitées par la 15d-PGJ$_2$ ou par la rosiglitazone, comme précédemment décrit. En cours et en fin d'expérimentation, des prélèvements sanguins ont été effectués afin de suivre la présence des levures dans le sang des souris. Aucun échantillon n'a montré la présence de levures dans le sang, signifiant ainsi qu'il n'y a eu aucune dissémination de l'infection fongique. Au terme du protocole, les souris ont été euthanasiées et les différents organes ont été prélevés stérilement en vue de quantifier le nombre de levures présentes dans les différents organes par CFU et PCR quantitative en temps réel.

Pour quantifier le nombre de levures présentes dans les différents organes par CFU, après homogénéisation de chaque tissu, plusieurs dilutions sont réalisées et ensemencées dans des boîtes de culture. Les boîtes sont incubées à 35°C pendant 24 à 48 heures et le nombre de colonies est alors compté.

Pour quantifier le nombre de levures présentes dans les différents organes par PCR, après homogénéisation des différents organes, l'ADN total est extrait par la méthode phénol/chloroforme et soumis à une Q-PCR en temps réel. La quantification d'ADN de *C. albicans* est réalisée sur automate LightCycler (ROCHE MOLECULAR BIOCHEMICALS, Suisse). Les amorces utilisées ont été choisies sur les régions conservées du gène codant pour l'ARNr 18S fongique. La détection se fait par hybridation de deux sondes marquées spécifiques de *C. albicans* (transfert de fluorescence).

Essai 8 : Stimulation de la phagocytose de *C. albicans* et de la production d'agents oxydants des macrophages par un traitement *in vivo* par la 15d-PGJ$_2$.

L'étude a été réalisée sur des souris immunocompétentes ou immunodéficientes de type RAG-2, (souris qui possèdent un déficit combiné sévère de l'immunité à médiation cellulaire et humorale, n'ont pas de lymphocytes B et T) traitées ou non, soit par le 15d-PGJ$_2$ soit par la rosiglitazone, puis infectées par *C. albicans* (culture, par la technique radiométrique, face à un challenge d'une heure avec *C. albicans,* dans un rapport 1: 1).

Le tableau 5, ci-après, fait état de la capacité de phagocytose des macrophages péritonéaux prélevés sur des souris SWISS ou RAG-2 infectées par *C. albicans,* traités ou non *in vivo* par la 15d-PGJ$_2$. Les résultats présentés dans ce tableau montrent que les macrophages extraits des souris SWISS et des souris RAG-2, traitées *in vivo,* soit par la 15d-PGJ$_2$ soit par la rosiglitazone, présentent une capacité de phagocytose beaucoup plus importante que les macrophages extraits des souris nom traitées *in vivo* (contrôle). Ainsi, l'administration par voie intra-péritonéale de la 15d-PGJ$_2$ ou de la rosiglitazone augmente la capacité des macrophages à internaliser *C. albicans*.

Tableau 5

| Traitements | Index de phagocytose | | |
|---|---|---|---|
| | Contrôle | 15d-PGJ$_2$ | rosiglitazone |
| SWISS | 42,97 $\pm$ 1,20 | 65,26 $\pm$ 2,11 | 60,51 $\pm$ 1,88 |
| RAG-2 | 59,82 $\pm$ 2,43 | 76,32 $\pm$ 1,89 | 72,48 $\pm$ 1,25 |

La capacité des macrophages à produire des agents oxydants a été ensuite déterminée, ainsi que la modulation de cette production par le traitement *in vivo* avec de la 15d-PGJ$_2$ ou de la rosiglitazone.

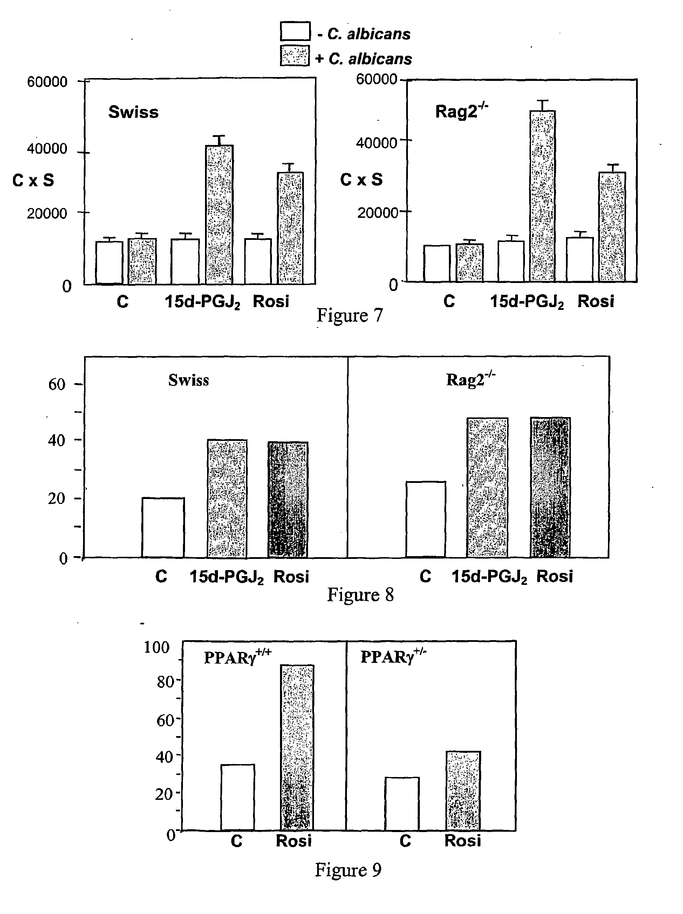

L'évaluation de la production d'agents oxydants par les macrophages, en présence ou en absence de la levure, a été déterminée sur des macrophages en culture par la technique de chimiluminescence. La figure 7 expose les résultats obtenus, en nombre de coups secondes (C x S) comptabilisés pendant 1 heure, pour chaque test.

La figure 7 montre que les macrophages extraits des souris SWISS et RAG-2, traitées *in vivo* par la 15d-PGJ$_2$ produisent la même quantité basale d'agents oxydants que les macrophages extraits des souris contrôles. Le traitement des souris avec la 15d-PGJ$_2$ n'induit donc pas une augmentation de la production des agents oxydants par les macrophages. Néanmoins, l'ajout des levures augmente la production d'agents oxydants.

Essai 9 : Amplification de l'expression des récepteurs mannose par un traitement *in vivo* avec la 15d-PGJ$_2$ ou avec la rosiglitazone.

Comme le récepteur mannose est impliqué dans la phagocytose de nombreux micro-organismes pathogènes comme en particulier *C. albicans,* l'expression de ce récepteur à la surface des macrophages extraits des souris traitées *in vivo,* par la 15d-PGJ$_2$ ou par la rosiglitazone, a été étudiée par cytométrie en flux.

La figure 8 montre que la quantité d'albumine bovine mannosylée marquée à la fluorescéine présente sur les macrophages est significativement augmentée quand les cellules sont extraites des souris traitées *in vivo* par la 15d-PGJ$_2$ ou par la rosiglitazone. Ceci suggère que le traitement des souris SWISS et RAG-2, avec la 15d-PGJ$_2$ ou avec la rosiglitazone, induit une augmentation de l'expression des récepteurs mannose à la surface des macrophages.

La figure 9 montre que l'induction du récepteur mannose par les ligands de PPAR$\gamma$ est largement diminuée dans des souris ayant un allèle nul pour le gène de ce récepteur nucléaire (souris hétérozygotes, PPAR$\gamma^{+/-}$) par rapport aux souris homozygotes (PPAR$\gamma^{+/+}$). Ainsi, l'effet des ligands de PPAR$\gamma$ est plus efficace sur des souris qui expriment le gène de PPAR$\gamma$ sur les deux allèles du chromosome ce qui suggère le rôle de PPAR$\gamma$ dans l'expression du récepteur mannose *in vivo.*

L'analyse de régression des résultats exposés à la figure 8 et au tableau 5 indique une association linéaire entre la quantité de MMR à la surface des macrophages et la capacité de phagocytose de ces cellules après traitement des souris, par la 15d-PGJ$_2$ ou par la rosiglitazone. Ceci démontre donc une vraie corrélation entre l'expression des récepteurs mannose influencée par les traitements et la capacité de ces cellules à internaliser *C. albicans.*

Essai 10 : Diminution de l'infection candidosique par un traitement *in vivo* avec la 15d-PGJ$_2$ ou avec la rosiglitazone.

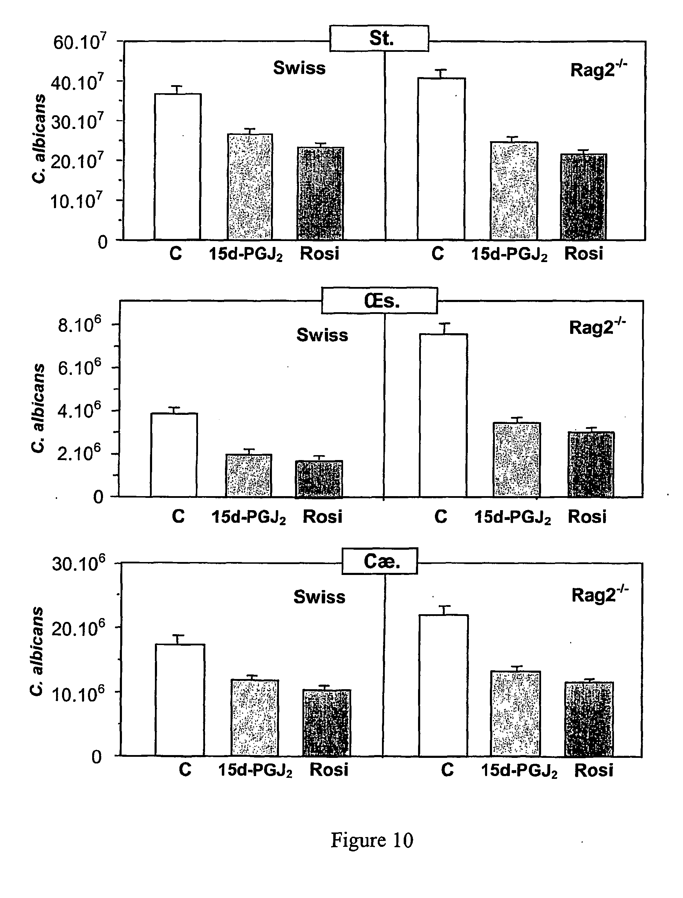

Dans une deuxième étape, il a été établi que les traitements modifiaient l'infection candidosique au niveau de plusieurs organes (oesophage, estomac, caecum, reins, foie). Pour ce faire, après infection par *C. albicans,* les souris SWISS et RAG-2 ont été traitées soit par la 15d-PGJ$_2$ soit par la rosiglitazone. En cours et en fin d'expérimentation, des prélèvements sanguins ont été effectués afin de suivre la présence des levures dans le sang des souris. Aucun échantillon n'a montré la présence de levures dans le sang, signifiant ainsi qu'il n'y a eu aucune dissémination de l'infection fongique. Au terme du protocole, les souris ont été euthanasiées et les différents organes ont été prélevés stérilement en vue de quantifier le nombre de levures présente dans les différents organes par CFU et PCR quantitative en temps réel. Les résultats obtenus sur l'oesophage (OEs.), l'estomac (St.), et le caecum (Cæ.), sont présentés à la figure 10.

Les deux techniques de quantification du nombre de levures montrent une diminution significative de la colonisation des différents organes chez les souris traitées, soit par la 15d-PGJ$_2$ soit par la rosiglitazone, par rapport aux souris non traitées (C). Au niveau du foie et des reins, aucune colonisation par la levure n'est observée montrant ainsi une absence de dissémination de l'infection fongique.

## Revendications

1. Procédé de fabrication d'une composition thérapeutique destinée au traitement préventif ou curatif d'une pathologie appartenant au groupe des pathologies induites par des micro-organismes d'origine bactérienne, fongique ou protozoaire, qui ont, sur la face extérieure de leurs parois, des polysaccharides et/ou des lipopolysaccharides ayant à leurs extrémités libres des motifs mannosylés et/ou fucosylés -à l'exception de *Mycobacterium avium-,* lesdits micro-organismes étant choisis *parmi : Streptococcus pneumoniae, Klebsellia pneumoniae, Cryptococcus neoformans, Mycobacterium tuberculosis, Candida albicans, Leishmania donovani, Pneumocystis carinii, Trypanosoma cruzi, Aspergillus fumigatus, Pseudomonas aeruginosas, Legionella pneumophilla,* **caractérisé en ce qu'**on utilise une quantité thérapeutiquement efficace d'au moins un ligand agoniste du récepteur nucléaire PPARγ à titre de facteur d'induction et/ou d'amplification de l'expression des récepteurs mannose des macrophages.

2. Procédé de fabrication d'une composition thérapeutique selon la revendication 1, **caractérisé en ce qu'**au moins un des ligands agonistes de PPARγ est choisi parmi le groupe des ligands naturels : la 15d-PGJ$_2$, les prostaglandines A1 et D2, les acides 9- et 13-hydoxyoctadécanoïques, les dérivés des LDL oxydées.

3. Procédé de fabrication d'une composition thérapeutique selon la revendication 1, **caractérisé en ce qu'**au moins un des ligands agonistes de PPARγ est choisi parmi le groupe des ligands synthétiques : la troglitazone, la rosiglitazone, la pioglitazone, la ciglitazone, l'englitazone, le GW 347845, le KRP-297, le JTT-501β, le SB 213068, le GI 262570, le GW 1929, le GW 7845, le GW 0207, le L-796449, l'indométhacine et l'ibuprofène.

4. Procédé de fabrication d'une composition thérapeutique selon l'une des revendications 1 à 3, **caractérisé en ce que** ladite composition thérapeutique est destinée à la médication de sujets immunodéprimés.

5. Procédé de stimulation de l'expression de récepteurs mannose de macrophages dans lequel, on traite *in vitro,* par une quantité efficace d'au moins un ligand agoniste de PPARγ, un milieu biologique extra-corporel comprenant des macrophages.

6. Procédé de stimulation selon la revendication 5, **caractérisé en ce qu'**au moins un des ligands agonistes de PPARγ est choisi parmi le groupe des ligands naturels : la 15d-PGJ$_2$, les prostaglandines A1 et D2, les acides 9- et 13-hydoxyoctadécanoïques, les dérivés des LDL oxydées.

7. Procédé de stimulation selon la revendication 5, **caractérisé en ce qu'**au moins un des ligands agonistes de PPARγ est choisi parmi le groupe des ligands synthétiques : la troglitazone, la rosiglitazone, la pioglitazone, la ciglitazone, l'englitazone, le GW 347845, le KRP-297, le JTT-501β, le SB 213068, le GI 262570, le GW 1929, le GW 7845, le GW 0207, le L-796449, l'indométhacine et l'ibuprofène.

8. Procédé d'élimination de micro-organismes choisis *parmi : Streptococcus pneumoniae, Klebsellia pneumoniae, Cryptococcus neoformans, Mycobacterium tuberculosis, Candida albicans, Leishmania donovani, Pneumocystis carinii, Trypanosoma cruzi, Aspergillus fumigatus, Pseudomonas aeruginosas, Legionella pneumophilla,* **caractérisé en ce qu'**on place *in vitro,* dans un milieu infecté par lesdits micro-organismes et au contact de macrophages, une quantité efficace d'au moins un ligand agoniste de PPARγ à titre de facteur d'induction et/ou d'amplification de l'expression des récepteurs mannose des macrophages.

9. Procédé d'élimination selon la revendication 8, **caractérisé en ce qu'**au moins un des ligands agonistes de PPARγ est choisi parmi le groupe des ligands naturels : la 15d-PGJ$_2$, les prostaglandines A1 et D2, les acides 9- et 13-hydoxyoctadécanoïques, les dérivés des LDL oxydées.

10. Procédé d'élimination selon la revendication 8, **caractérisé en ce qu'**au moins un des ligands agonistes de PPARγ est choisi parmi le groupe des ligands synthétiques : la troglitazone, la rosiglitazone, la pioglitazone, la ciglitazone, l'englitazone, le GW 347845, le KRP-297, le JTT-501β, le SB 213068, le GI 262570, le GW 1929, le GW 7845, le GW 0207, le L-796449, l'indométhacine et l'ibuprofène.

## Claims

1. Process for the preparation of a therapeutic composition for preventive or curative treatment of a pathology belonging to the group of pathologies induced by microorganisms of bacterial, fungal or protozoal origin which have, on the outer surface of their walls, polysaccharides and/or lipopolysaccharides having at their free ends mannosylated and/or fucosylated motifs - excluding Mycobacterium avium - the said microorganisms being chosen from: Streptococcus pneumoniae, Klebsiella pneumoniae, Cryptococcus neoformans, Mycobacterium tuberculosis, Candida albicans, Leishmania donovani, Pneumocystis carinii, Trypanosoma cruzi, Aspergillus fumigatus, Pseudomonas aeruginosa, Legionella pneumophila, **characterized in that** a therapeutically effective amount of at least one agonist ligand of the PPARγ nuclear receptor is used as a factor for induction and/or amplification of the expression of macrophage mannose receptors.

2. Process for the preparation of a therapeutic composition according to claim 1, **characterized in that** at least one of the agonist ligands of PPARγ is chosen from the group of natural ligands: 15d-PGJ2, prostaglandins A1 and D2, 9- and 13-hydroxyoctadecanoic acid and oxidized derivatives of LDL.

3. Process for the preparation of a therapeutic composition according to claim 1, **characterized in that** at least one of the agonist ligands of PPARγ is chosen from the group of synthetic ligands: troglitazone, rosiglitazone, pioglitazone, ciglitazone, englitazone, GW 347845, KRP-297, JTT-501a, SB 213068, GI 262570, GW 1929, GW 7845, GW 0207, L-796449, indometacin and ibuprofen.

4. Process for the preparation of a therapeutic composition according to one of claims 1 to 3, **characterized in that** the said therapeutic composition is for medication of immunodepressed subjects.

5. Method for stimulation of the expression of macrophage mannose receptors, in which an extracorporeal biological medium containing macrophages is treated in vitro with an effective amount of at least one agonist ligand of PPARγ.

6. Method for stimulation according to claim 5, **characterized in that** at least one of the agonist ligands of PPARγ is chosen from the group of natural ligands: 15d-PGJ2, prostaglandins A1 and D2, 9- and 13-hydroxyoctadecanoic acid and oxidized derivatives of LDL.

7. Method for stimulation according to claim 5, **characterized in that** at least one of the agonist ligands of PPARγ is chosen from the group of synthetic ligands: troglitazone, rosiglitazone, pioglitazone, ciglitazone, englitazone, GW 347845, KRP-297, JTT-501β, SB 213068, GI 262570, GW 1929, GW 7845, GW 0207, L-796449, indometacin and ibuprofen.

8. Method for elimination of microorganisms chosen from: Streptococcus pneumoniae, Klebsiella pneumoniae, Cryptococcus neoformans, Mycobacterium tuberculosis, Candida albicans, Leishmania donovani, Pneumocystis carinii, Trypanosoma cruzi, Aspergillus fumigatus, Pseudomonas aeruginosa, Legionella pneumophila, **characterized in that** an effective amount of at least one agonist ligand of PPARγ, as a factor for induction and/or amplification of the expression of macrophage mannose receptors, is placed in vitro in a medium infected by the said microorganisms and in contact with the macrophages.

9. Method for elimination according to claim 8, **characterized in that** at least one of the agonist ligands of PPARγ is chosen from the group of natural ligands: 15d-PGJ2, prostaglandins A1 and D2, 9- and 13-hydroxyoctadecanoic acid and oxidized derivatives of LDL.

10. Method for elimination according to claim 8, **characterized in that** at least one of the agonist ligands of PPARγ is chosen from the group of synthetic ligands: troglitazone, rosiglitazone, pioglitazone, ciglitazone, englitazone, GW 347845, KRP-297, JTT-501β, SB 213068, GI 262570, GW 1929, GW 7845, GW 0207, L-796449, indometacin and ibuprofen.

## Patentansprüche

1. Herstellungsverfahren einer therapeutischen Verbindung, die für die präventive oder kurative Behandlung einer Pathologie bestimmt ist, die zur Gruppe der durch Mikroorganismen bakteriellen, Pilz- oder Protozoon-Ursprungs induzierten Pathologien gehört, die auf der äußeren Seite ihrer Wände Polysaccharide und / oder Lipopolysaccharide haben, die an ihren freien Enden mannosylierte und / oder fucosylierte Motive haben - mit Ausnahme von Mycobacterium avium -, wobei die genannten Mikroorganismen ausgewählt sind aus: *Streptococcus Pneumoniae, Klebsellia Pneumoniae, Cryptococcus Neoformans, Mycobacterium Tuberculosis, Candida Albicans, Leishmania Donovani, Pneumocystis Carinii, Trypanosoma Cruzi, Aspergillus Fumigatus, Pseudomonas Aeruginosas, Legionella Pneumophilla,* **dadurch gekennzeichnet, dass** eine therapeutisch wirksame Menge, aus wenigstens einem agonistischen Liganden des Kern-Rezeptors PPARγ als Induktions- und / oder Amplifikationsfaktor der Expression der Mannose-Rezeptoren der Makrophagen verwendet wird.

2. Herstellungsverfahren einer therapeutischen Verbindung gemäß Anspruch 1, **dadurch gekennzeichnet, dass** wenigstens einer der agonistischen Liganden von PPARγ aus der Gruppe der natürlichen Liganden ausgewählt ist: dem 15d-PGJ$_2$, den Prostaglandinen A1 und D2, den 9-und 13-Hydroxyoktadecanoid-Säuren, den Derivaten der oxidierten LDL.

3. Herstellungsverfahren einer therapeutischen Verbindung gemäß Anspruch 1, **dadurch gekennzeichnet, dass** wenigstens einer der agonistischen Liganden von PPARγ aus der Gruppe der synthetischen Liganden ausgewählt ist: dem Troglitazon, dem Rosiglitazon, dem Pioglitazon, dem Ciglitazon, dem Englitazon, dem GW 347845, dem KRP-297, dem JTT-501β, dem SB 213068, dem GI 262570, dem GW 1929, dem GW 7845, dem GW 0207, dem L-796449, dem Indomethacin und dem Ibuprofen.

4. Herstellungsverfahren einer therapeutischen Verbindung gemäß Anspruch 1 bis 3, **dadurch gekennzeichnet, dass** die genannte therapeutische Verbindung zur Medikation von immundeprimierten Patienten bestimmt ist.

5. Stimulationsverfahren der Expression von Mannoserezeptoren von Makrophagen, bei dem *in vitro* durch eine wirksame Menge von wenigstens einem agonistischen Liganden von PPARγ ein biologisches, außerkörperliches Milieu behandelt wird, das Makrophagen umfasst.

6. Stimulationsverfahren gemäß Anspruch 5, **dadurch gekennzeichnet, dass** wenigstens einer der agonistischen Liganden von PPARγ aus der Gruppe der natürlichen Liganden ausgewählt ist: dem 15d-PGJ$_2$, den Prostaglandinen A1 und D2, den 9- und 13-Hydroxyoktadecanoidsäuren, den Derivaten der oxidierten LDL.

7. Stimulationsverfahren gemäß Anspruch 5, **dadurch gekennzeichnet, dass** wenigstens einer der agonistischen Liganden von PPARγ aus der Gruppe der synthetischen Liganden ausgewählt ist: dem Troglitazon, dem Rosiglitazon, dem Pioglitazon, dem Ciglitazon, dem Englitazon, dem GW 347845, dem KRP-297, dem JTT-501β, dem SB 213068, dem GI 262570, dem GW 1929, dem GW 7845, dem GW 0207, dem L-796449, dem Indomethacin und dem Ibuprofen.

8. Eliminationsverfahren von Mikroorganismen, die ausgewählt sind aus: *Streptococcus Pneumoniae, Klebsellia Pneumoniae, Cryptococcus Neoformans, Mycobacterium Tuberculosis, Candida Albicans, Leishmania Donovani, Pneumocystis Carinii, Trypanosoma Cruzi, Aspergillus Fumigatus, Pseudomonas Aeruginosas, Legionella Pneumophilla,* **dadurch gekennzeichnet, dass** in vitro in einem von den genannten Mikroorganismen infizierten und mit Makrophagen in Kontakt stehendem Milieu eine wirksame Menge von wenigstens einem agonistischen Liganden von PPARγ als Induktions- und / oder Amplifikationsfaktor der Expression der Mannoserezeptoren der Makrophagen platziert wird.

9. Eliminationsverfahren gemäß Anspruch 8, **dadurch gekennzeichnet, dass** wenigstens einer der agonistischen Liganden von PPARγ aus der Gruppe der natürlichen Liganden ausgewählt ist: dem 15d-PGJ$_2$, den Prostaglandinen A1 und D2, den 9- und 13-Hydroxyoktadecanoidsäuren, den Derivaten der oxidierten LDL.

10. Eliminationsverfahren gemäß Anspruch 8, **dadurch gekennzeichnet, dass** wenigstens einer der agonistischen Liganden von PPARγ aus der Gruppe der synthetischen Liganden ausgewählt ist: dem Troglitazon, dem Rosiglitazon, dem Pioglitazon, dem Ciglitazon, dem Englitazon, dem GW 347845, dem KRP-297, dem JTT-501β, dem SB 213068, dem GI 262570, dem GW 1929, dem GW 7845, dem GW 0207, dem L-796449, dem Indomethacin und dem Ibuprofen.

Figure 1

Figure 2

Figure 3

Figure 4

Figure 5

Figure 6

Figure 7

Figure 8

Figure 9

Figure 10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03059271 A

**Littérature non-brevet citée dans la description**

- **Vamecq J. ; Latruffe N.** *The Lancet,* 1999, vol. 354, 141-148
- **Coste A. et al.** *Immunity,* 2003, vol. 19, 329-339
- **Forman B.M. et al.** *Cell,* 1995, vol. 83, 803-812
- **Yamamoto et al.** *Infect Immun,* 1997, vol. 65, 1077-82
- **Capacciolis et al.** *Biochem Biophys Res Commun,* 1998, vol. 197, 818-25
- **Coste A. et al.** *J. Antimicrobial Chemotherapy,* 2002, vol. 49, 731-740
- **Bureau et al.** *J. Biol. Chem.,* 2001, vol. 276, 23077-23083